(19) Europäisches Patentamt
European Patent Office
Office européen des brevets

(11) EP 1 625 147 B1

(12) **EUROPEAN PATENT SPECIFICATION**

(45) Date of publication and mention of the grant of the patent:
**23.01.2008 Bulletin 2008/04**

(21) Application number: **04733570.8**

(22) Date of filing: **18.05.2004**

(51) Int Cl.:
***C07K 1/16*** *(2006.01)*

(86) International application number:
**PCT/EP2004/005401**

(87) International publication number:
**WO 2004/104025 (02.12.2004 Gazette 2004/49)**

# (54) METHOD OF CHROMATOGRAPHIC ANALYSIS OF A PROTEIN SOLUTION

VERFAHREN ZUR CHROMATOGRAPHISCHEN ANALYSE EINER PROTEINLÖSUNG

PROCEDE D'ANALYSE CHROMATOGRAPHIQUE D'UNE SOLUTION PROTEIQUE

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **21.05.2003 EP 03101458**

(43) Date of publication of application:
**15.02.2006 Bulletin 2006/07**

(73) Proprietor: **ARES TRADING S.A.**
**1170 Aubonne (CH)**

(72) Inventors:
- **ARPAIA, Giuseppe**
  **I-00184 Roma (IT)**
- **BERARDI, Marco**
  **I-00040 Ciampino Roma (IT)**
- **CHAVEZ, Enrico**
  **CH-1110 Morges (CH)**
- **DRIEBERGEN, Reinoud**
  **1212 Grand Lancy (CH)**
- **GIARTOSIO, Carlo, Emanuele**
  **I-00196 Roma (IT)**
- **LEPAGE, Pierre**
  **F-01220 Sauverny (FR)**

(74) Representative: **Merck Serono International S.A.**
**Intellectual Property**
**Intellectual Property Department**
**9, chemin des Mines**
**1202 Geneva (CH)**

(56) References cited:
**EP-A- 0 530 937**
**DE-A- 3 917 949**
**US-A1- 2003 021 765**
**US-B1- 6 414 123**

- **MANOHAR KATAKAM ET AL: "Effect of Surfactants on the Physical Stability of Recombinant Human Growth Hormone" JOURNAL OF PHARMACEUTICAL SCIENCES, AMERICAN PHARMACEUTICAL ASSOCIATION. WASHINGTON, US, vol. 84, no. 6, June 1995 (1995-06), pages 713-716, XP002114576 ISSN: 0022-3549**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of the invention

This invention relates to methods for the analysis of proteins.

More specifically, it relates to the analysis of proteins by chromatographic methods (e.g. HPLC). By "analysis" of proteins is meant here the quantitative determination of a protein.

### Background of the invention

Proteins in pharmaceutical products must be analysed to quantify the protein and to ensure purity. This permits correct and reproducible dosing to the patient.

Analytical techniques are necessary to quantify the pharmaceutical protein as well as impurities, aggregates, and degradation products. In the case of multimeric proteins, such as dimers, analytical techniques are required to detect the presence and extent (i.e. quantity) of dissociation.
Such analytical techniques should be precise (i.e. the degree of variation when the same sample is tested multiple times should be low), and accurate (i.e. the measured value should be as close as possible to the actual value). Reproducibility is also important.

An example of an analytical assay that may be used with proteins is size exclusion chromatography (SEC), in which a protein in aqueous solution is passed over a solid or gel phase that separates mixtures of protein by differences in their molecular weight. The resulting chromatogram shows one or more peaks associated with the protein(s) in a sample, which may be identified by molecular weight. The area under a peak associated with a given protein can be used to quantify the amount of that protein in the sample. The shape of the peak may be used to assess purity.

Another example of an analytical assay that may be used with proteins is high performance liquid chromatography (HPLC), in particular reverse phase high performance liquid chromatography (RP-HPLC). A sample containing the pharmaceutical protein is passed through a column which separates the protein from any impurities. The protein and any impurities elute as peaks on a chromatogram. As with SEC, the area under a peak associated with a given protein can be used to quantify the amount of that protein in the sample. The shape of the peak may be used to assess purity.

In chromatographic methods, such as those mentioned above, the protein must be dissolved in an aqueous solvent and diluted to an extent acceptable for the chromatographic system used. During handling of the aqueous protein solution, the risk exists that part of the protein is lost by adsorption to handling and containment equipment, such as glass or plastic walls of capillaries, test-tubes, beakers, syringes, etc., making quantitation of protein difficult. The adsorption of protein leads to variations in the results that detract from the assay precision, accuracy and reproducibility

Surfactants have been used in the purification of proteins by SEC, and in assays in which the molecular weight of a new protein is determined, see for example EP 0 530 937 and DE 39 17 949.

Katakam et al., Journal of Pharmaceutical Sciences 84: 713-716 (1995) describe a study on the physical stability of a human growth hormone formulation upon exposure to air/water interfaces. Further, the influence of non-ionic surfactants, such as pluronic F68, on the stability was investigated. Aggregate formation was analysed by SEC-HPLC. This chromatographic analysis allowed to draw conclusions on the nature of the aggregates, namely the fact that they were non-covalent.

It would be desirable to have a chromatographic method of protein analysis for quantifying protein and/or assessing purity, in which assay precision, accuracy and reproducibility are improved by avoiding variations due to protein adsorption.

### Summary of the invention

It has now been discovered that a surfactant of the class of Poloxamers avoids protein loss and at the same time does not interfere with the chromatographic analysis.

In a first aspect, the invention provides a method of chromatographic analysis of a protein in a sample for quantifying the protein, wherein the method comprises:

1) a step of preparing the protein sample to contain a poloxamer;
2) a step of performing a chromatography; and
3) a step of data manipulation to determine the quantity of the protein, wherein the quantity of the protein is determined using data from calibration with a standard.

In another aspect, the invention provides the use of a poloxamer in chromatographic analysis for quantifying

the protein in a protein sample.

**Detailed description of the invention**

The inventors have found that by including a Poloxamer in a protein solution to be assayed for protein content, protein adsorption is decreased, leading to increased assay precision, accuracy and reproducibility.

in the context of the present application, the term "analysis" is meant to encompass an analytical process whereby the quantity (e.g. concentration) of a protein in a sample is determined. The method of the invention comprises a method for the analysis of the quantity of a protein in a sample, the method comprising a step of preparing the protein sample to contain a Poloxamer, and performing a step of chromatography, preferably a step of SEC or RP-HPLC. The step of chromatography is followed by a step of data manipulation to determine the quantity of the protein. The quantity of protein is determined using data from calibration with a standard. Calibration may be carried out before or after analysis of the sample.

Poloxamers are block copolymers made of poly(oxyethylene)-poly(oxypropylene) blocks with M.W. ranges from 1,000 to > 16,000. Their main characteristic is that the poly(oxyethylene) segments are hydrophilic and the poly (oxypropylene) segments hydrophobic. These substances behave as non-ionic surfactants and are generally known with the commercial name "Pluronics".

Many grades of Pluronics at various Molecular Weight and concentration ranges can be used in accordance with this invention.

As mentioned above, the Poloxamer (Pluronic) surfactants are block copolymers of ethylene oxide (EO) and propylene oxide (PO). The propylene oxide block (PO) is sandwiched between two ethylene oxide (EO) blocks.

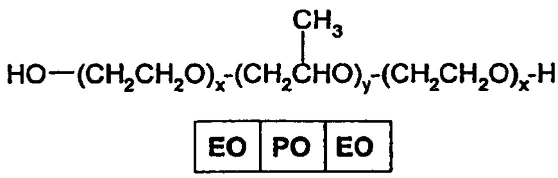

Pluronic surfactants are synthesised in a two-step process:

1. A hydrophobe of the desired molecular weight is created by the controlled addition of propylene oxide to the two hydroxyl groups of propylene glycol; and

2. Ethylene oxide is added to sandwich the hydrophobe between hydrophilic groups.

In Pluronic® F77, the percentage of polyoxyethylene (hydrophile) is 70%, and the molecular weight of the hydrophobe (polyoxypropylene) is approximately 2,306 Da.

In Pluronic F87, the percentage of polyoxyethylene (hydrophile) is 70%, and the molecular weight of the hydrophobe (polyoxypropylene) is approximately 2,644 Da.

In Pluronic F88, the percentage of polyoxyethylene (hydrophile) is 80%, and the molecular weight of the hydrophobe (polyoxypropylene) is approximately 2,644 Da.

In Pluronic F68, the percentage of polyoxyethylene (hydrophile) is 80%, and the molecular weight of the hydrophobe (polyoxypropylene) is approximately 1,967 Da.

Typical properties of Pluronic F77 are listed below:

Average Molecular Weight: 6600;
Melt/pour point: 48°C ;
Physical Form @ 20°C : solid;
Viscosity (Brookfield) cps: 480 [liquids at 25°C, pastes at 60°C and solids at 77°C];
Surface tension, dynes/cm @ 25°C;

0.1 % Conc. : 47.0
0.01% Conc. : 49.3
0.001% Conc.: 52.8

Interfacial tension, dynes/cm @ 25°C vs. Nujol;

0.1% Conc. : 17.7

0.01% Conc. : 20.8

0.01% Conc. : 25.5

Draves Wetting, Seconds 25°C

1.0% Conc.: > 360

0.1 % Conc.: > 360

Foam Height

Ross Miles, 0.1 %, mm @ 50°C: 100
Ross Miles, 0.1 %, mm @ 26°C: 47
Dynamic, 0.1%, mm @ 400 ml/min: > 600

Cloud point in aqueous solution, °C

1% Conc.: >100
10% Conc.: >100

HLB (hydrophile-lipophile balance): 25

Typical properties of Pluronic F87 are listed below:

Average Molecular Weight: 7700;
Melt/pour point: 49°C ;
Physical Form @ 20°C : solid;
Viscosity (Brookfield) cps: 700 [liquids at 25°C, pastes at 60°C and solids at 77°C];
Surface tension, dynes/cm @ 25°C;

0.1 % Conc. : 44.0
0.01 % Conc. : 47.0
0.001 % Conc.: 50.2

Interfacial tension, dynes/cm @ 25°C vs Nujol;

0.1% Conc.: 17.4
0.01% Conc. : 20.3
0.01 % Conc. : 23.3

Draves Wetting, Seconds 25°C

1.0% Conc.: > 360
0.1% Conc.: > 360

Foam Height

Ross Miles, 0.1%, mm @ 50°C: 80
Ross Miles, 0.1%, mm @ 26°C: 37
Dynamic, 0.1 %, mm @ 400 ml/min: > 600

Cloud point in aqueous solution, °C

1% Conc.: >100
10% Conc.: >100

HLB (hydrophile-lipophile balance): 24

Typical properties of Pluronic F88 are listed below:

Average Molecular Weight: 11400;
Melt/pour point: 54°C ;
Physical Form @ 20°C : solid;
Viscosity (Brookfield) cps: 2300 [liquids at 25°C, pastes at 60°C and solids at 77°C];
Surface tension, dynes/cm @ 25°C;

0.1 % Conc. : 48.5
0.01% Conc. : 52.6
0.001% Conc.: 55.7

Interfacial tension, dynes/cm @ 25°C vs Nujol;

0.1 % Conc. : 20.5
0.01 % Conc. : 23.3
0.01 % Conc. : 27.0

Draves Wetting, Seconds 25°C

1.0% Conc.: > 360
0.1 % Conc.:> 360

Foam Height

Ross Miles, 0.1 %, mm @ 50°C: 80
Ross Miles, 0.1%, mm @ 26°C: 37
Dynamic, 0.1 %, mm @ 400 ml/min: > 600

Cloud point in aqueous solution, °C

1% Conc.: >100
10% Conc.: >100

HLB (hydrophile-lipophile balance): 28

Typical properties of Pluronic F68 are listed below:

Average Molecular Weight: 8400;
Melt/pour point: 52°C ;
Physical Form @ 20°C : solid;
Viscosity (Brookfield) cps: 1000 [liquids at 25°C, pastes at 60°C and solids at 77°C];
Surface tension, dynes/cm @ 25°C;

0.1 % Conc. : 50.3
0.01% Conc. : 51.2
0.001% Conc.: 53.6

Interfacial tension, dynes/cm @ 25°C vs Nujol;

0.1% Conc. : 19:8
0.01% Conc. : 24.0
0.01 % Conc. : 26.0

Draves Wetting, Seconds 25°C

1.0% Conc.: > 360
0.1% Conc.: > 360

Foam Height

Ross Miles, 0.1 %, mm @ 50°C: 35
Ross Miles, 0.1%. mm @ 26°C: 40
Dynamic, 0.1 %, mm @ 400 ml/min: > 600

Cloud point in aqueous solution, °C

1% Conc.: >100
10% Conc.: >100

HLB (hydrophile-lipophile balance): 29

Other polymers having properties similar to those listed above may also be used in the methods of the invention. The preferred surfactant is Pluronic F68 (Poloxamer 188), and surfactants having similar properties.

The method according to the invention can be used essentially with any protein, for example insulin, etanercept, Factor VIII, growth hormone (Somatotropin), antibodies (such as infliximab), leukaemia inhibitory factor (LIF, enfilermin), an interleukin, such as interleukin-6 (Atexakin alpha), tumour necrosis factor binding protein (TBP-1, Onercept), interleukin-18 binding protein (IL-18 BP, Tadekin), anti-CD11a (Efalizumab). In a preferred embodiment, the protein is a glycoprotein, such as erythropoietin (EPO), darbepoietin alfa, human protein C, interferons (such as interferon beta 1a, 1b, particularly preferably interferon-beta-1a), alpha galactosidase A. Preferably the protein is a dimeric protein, i.e. composed of subunits (including heterodimeric), particularly preferably a dimeric or heterodimeric glycoprotein, for example thyroid-stimulating hormone (TSH), and the gonadotrophins: follicle-stimulating hormone (FSH), luteinising hormone (LH), and chorionic gonadotrophin (CG). Preferably these proteins are human proteins.

In the Examples which follow, Pluronic F68 (Poloxamer 188) is used at the concentration of 100 μg /ml in ultra-pure water. It is however understood that the use of different grades of Pluronic and different concentrations of the same is encompassed by the present invention.

**EXAMPLE1**

**Improved sample preparation in the SEC method of quantitative determination of FSH**

The purpose of this study was to qualify an improved sample preparation in the Size Exclusion Chromatography (SEC) method for protein content in a preparation containing rec-FSH (Fertinex, in this case Fertinex 75 IU).

The modification implemented was the use of a solution including 100 μg/ml Pluronic F68 in ultra-pure water for preparation of all protein solutions (sample, control sample and standard) in order to control losses of protein due to adsorption.

Samples consisting of mixtures of heterodimeric FSH (FSH is composed of an α-subunit and a β-subunit) and dissociated FSH were prepared and analysed. The method allowed the quantitation of heterodimeric FSH and free subunits.

This study shows that a single point calibration curve using drug product reference standard is suitable to determine protein content with a good total precision of 2.0% and that the method is linear within the range tested (26.6 to 160 μg/ml).

In addition, both Waters and Varian HPLC systems can be used as shown during the study. The difference in the mean protein content results of all batches is lower than the total precision of the method.

It is important to emphasize that this change did not have any impact on the original SEC method itself.

More specifically, the modification of the sample and standard preparations were as follows:

- Use of a 100 μg/ml Pluronic F68 in ultra-pure water for the preparation of the samples and reference standard.
- Blank solution used through the analytical sequence is the 100 μg/ml Pluronic F68 solution.
- The preparation for SST control sample as well as control samples injected over the analytical session are performed by reconstituting and pooling enough ampoules to allow the injections of the system suitability as well as for the controls which are injected throughout the analytical session.

## Materials and equipment

### Materials

Control sample: highly purified urinary FSH (u-FSH-HP) batch 17304010 Test samples: 75 IU u-FSH-HP batch nos. 17301010, 17315040 and 17318040.

### Equipment

HPLC pump mod. 600E or 626 [Waters]
UV detector mod. 486 or 996 [Waters]
Column: Progel TSK G2000 SW 60 cm X 7.5 mm [Supelco]

### Procedure

The column was conditioned for an hour with the mobile phase (phosphate buffer 0.1 M; $Na_2SO_4$ 0.1 M, pH 6.70), at a flow rate of 0.70 ml/minute. The column was maintained at approximately 4°C throughout the analysis.
For analysis, the following solutions were used:

**Standard solution:** a solution of highly purified FSH (0.041 μg/μl) was prepared with Pluronic F68 (100 μg/ml).

**Sample solutions:** the sample solutions were prepared to have varying amounts of heterodimeric FSH (Fertinex) and dissociated FSH, in a solution containing Pluronic F68 (100 μg/ml).

**Control solutions:** control solution was prepared using FSH dissolved in a solution containing. Pluronic F68 (100 μg/ml).

The standard and sample solutions were injected (100 μl), and the column was eluted at a constant flow rate of 0.70 ml/min. Detection was by UV absorption at 214 nm. Area under the peaks was used to determine heterodimeric FSH and the respective FSH subunits.

### Results

The precision of an analytical method expresses the closeness of agreement (degree of scatter) between a series of measurements obtained from multiple sampling of the same homogeneous sample under the prescribed conditions. Precision may be considered at three levels : repeatability (or intra-assay precision), intermediate precision and reproducibility. During this study, repeatability, intermediate precision as well as reproducibility of the assay were addressed.
In five independent analytical sessions, three Fertinex drug products batches were quantified against the standard (calibration curve).
With the results of Fertinex 75 IU presented in Table 1, an analysis of variance (ANOVA) Nested design was used to estimate the repeatability (or intra-assay precision), intermediate precision and reproducibility (total precision) of the analytical method. The total number of results under study was 45. The results obtained were as follows:

- Repeatability (or intra-assay precision): 1.3%
- Intermediate precision: 1.0 %
- Reproducibility (total precision): 1.6%

Overall results are tabulated in the following Table 1:

**Table 1: Protein content results (μg/ampoule) obtained with calibration curve standard**

| Batch number | Run 1 | Run 2 | Run 3 | Run 4 | Run 5 |
|---|---|---|---|---|---|
| 17301010 | 6,41 | 6,42 | 6,32 | 6,56 | 6,41 |
| 17315040 | 6,13 | 6,06 | 5,94 | 6,11 | 6,15 |

(continued)

| Batch number | Run 1 | Run 2 | Run 3 | Run 4 | Run 5 |
|---|---|---|---|---|---|
| 17318040 | 5,54 | 5,58 | 5,49 | 5,59 | 5,51 |
| Control sample | 6,00 | 6,18 | 6,04 | 6,21 | 6,07 |

The standard results obtained during the precision and ruggedness studied were used to address the linearity and range of the analytical method. The approach for the statistical analysis was to check variance homogeneity (Cochran C and Bartlett tests), lack of fit and perform regression analysis (correlation coefficient). Run 1 to Run 5 were performed with a Waters systems whereas Run 6 to Run 8 were performed with a Varian system.

**Table 2: Statistical results for linearity**

| | Run 1 | Run 2 | Run 3 | Run 4 | Run 5 | Run 6 | Run 7 | Run 8 |
|---|---|---|---|---|---|---|---|---|
| Cochran's C test | 0,99 | 0,98 | 0,99 | 0,99 | 0,99 | 0,98 | 0,96 | 0,98 |
| Bartlett's test | 0,99 | 0,98 | 0,99 | 0,99 | 0,99 | 0,98 | 0,96 | 0,98 |
| Lack of fit | 0,97 | 0,18 | 0,83 | 0,92 | 0,81 | 0,89 | 0,03 | 0,07 |
| Slope | 1,0058 | 0,9926 | 1,0028 | 1,0066 | 0,9940 | 0,9903 | 1,0202 | 0,9888 |
| Intercept | 6,1557 | 6,2238 | 6,1574 | 6,2099 | 6,2249 | 5,5055 | 5,7437 | 5,5214 |
| Correlation coeff. | 1,0000 | 0,9999 | 0,9999 | 0,9999 | 1,0000 | 0,9999 | 0,9998 | 0,9999 |

As can be seen, the p-values of Cochran C and Bartlett tests are always greater than 0.05 which means that there is no statistically significant difference amongst the standard deviations at 95% confidence level and a correlation coefficient higher than 0.9980 is always met.

Lack of fit to determine whether linear regression is an adequate model to describe the observed data was performed. Based on this statistical test, linear regression resulted to be the best model to describe the data even if the p-value of Run 7 is not greater than 0.05. For that particular analytical session, the linear regression is still the best model with a 99.96% fit. Other models, such as square root and exponential models were tested and did not show a better fit of the data observed.

**EXAMPLE 2**

**Improved sample preparation and spiking procedure in the SEC method of purity determination of FSH**

In a spiking experiment, known amounts of either heterodimeric FSH or dissociated FSH were added to a sample of FSH ("spiking"). The resulting peaks for heterodimeric and/or dissociated subunits were evaluated for % recovery and for % purity.

The modification implemented in the analytical method is the use of a solution including 100 μg/ml Pluronic F68 in ultra-pure water for preparation of all protein solutions (sample, spike solution) to control losses of protein due to adsorption. The modifications were made specifically to increase the precision of the method, without impacting the chromatography of the method, to enable a more precise and accurate purity determination.

In the frame of this study, determination of the precision of the method was also investigated. The precision of the method (Total precision 1.8%) was slightly improved when compared to the precision of the method observed during validation of the analytical method without introduction of Pluronic F68 (Precision 2%). Routine recovery of the spike solutions at 100% indicates a good accuracy.

An additional spiking experiment was performed to further determine accuracy of the method. It was observed that different area under the peak is obtained for the spiking solution with and without Pluronic F68. The area of the spiking solution with Pluronic F68 in the sample preparation is approximately two times greater than the spiking solution prepared without the use of Pluronic F68.

It is believed that the different areas observed are due to adsorption of the dissociated sub-units on the polypropylene material used for sample preparation.

It can be seen in the following Tables 3 and 4 that the recovery of spiked free sub-units of FSH is within the range 95% - 105%. In addition it can also be seen that the precision [reported as coefficient of variability (CV%)] over the five runs are ranging from 1.0% to 1.5% for both purity and recovery of spike. The lower the CV%, the lesser the

variability between runs.

This is a substantial improvement over samples prepared without Pluronic F68.

**Table 3: %Recovery of spike results**

| Batch# | Run 1 | Run 2 | Run 3 | Run 4 | Run 5 | Mean | CV % |
|---|---|---|---|---|---|---|---|
| **17343109** | 98,0% | 98,1% | 100,3% | 99,6% | 99,4% | 99,1% | 1,0% |
| **17349129** | 98,9% | 99,6% | 102,1% | 100,7% | 102,2% | 100,7% | 1,5% |
| **17301010** | 99,8% | 100,7% | 102,0% | 99,2% | 99,8% | 100,3% | 1,1% |
| **17304010** | 99,8% | 102,4% | 101,6% | 101,0% | 99,9% | 100,9% | 1,1% |

**Table 4: %Purity of spike results**

| Batch# | Run 1 | Run 2 | Run 3 | Run 4 | Run 5 | Mean | CV % |
|---|---|---|---|---|---|---|---|
| **17343109** | 97,0% | 94,4% | 93,9% | 96,3% | 96,3% | 95,6% | 1,4% |
| **17349129** | 96,2% | 93,4% | 93,3% | 95,5% | 94,4% | 94,6% | 1,4% |
| **17301010** | 96,8% | 95,0% | 94,1% | 96,9% | 97,2% | 96,0% | 1,4% |
| **17304010** | 95,9% | 93,6% | 94,0% | 95,9% | 97,0% | 95,3% | 1,5% |

In the frame of another study protocol, the analysis of drug product batches with and without the use of Pluronic F68 was performed. One of the results is that the area under the peak of the spiking solution (dissociated r-hFSH) is approximately multiplied by two with the introduction of Pluronic F68. This phenomenon is most probably due to adsorption of free sub-units on the material used during sample preparation if Pluronic F68 is not present. To determine if this increase of area has an impact on the analysis, a spiking experiment at three levels without and with Pluronic F68 was performed. The following samples were tested in duplicate:

**1.Sample preparation with Pluronic F68**

- Sample without spiking solution;
- Sample spiked with dissociated r-hFSH (2 μg by injection);
- Spiking solution (2 μg dissociated r-hFSH by injection);
- Sample spiked with dissociated r-hFSH (1.5 μg by injection);
- Spiking solution (1.5 μg dissociated r-hFSH by injection);
- Sample spiked with dissociated r-hFSH (1 μg by injection);
- Spiking solution (1μg dissociated r-FSH by injection).

The results are presented in table 5:

**Table 5: Results of sample preparations using Pluronic F68: area under peak**

| Spiking level | Area of spiking solution | % Recovery of areas | % Purity | % Recovery of spike |
|---|---|---|---|---|
| 100% (2 μg/inj) | 2037915 | N/A | 95% | 101% |
| 75% (1.5 μg/inj) | 1545486 | 76% | 94% | 101% |
| 50% (1 μg/inj) | 1001055 | 49% | 94% | 102% |

From the above table, it can be seen that the addition of Pluronic F68 gives good results in term of % recovery of spike, being close to the theoretical recovery (100%) and % Recovery of areas. This latter parameter is calculated by dividing the area of the spiking solution at the defined spiking level by the area of the spiking solution at 100%. The result (i.e. 76% for spiking with 1.5 μg/injection) is compared with the theoretical spiking level performed (i.e. 75%).

**2. Sample preparation without Pluronic F68**

The following samples were tested in duplicate:

- Sample without spiking solution;
- Sample spiked with dissociated r-hFSH (2 μg by injection);
- Spiking solution (2 μg dissociated r-hFSH by injection);
- Sample spiked with dissociated r-hFSH (3 μg by injection) ;
- Spiking solution (3 μg dissociated r-hFSH by injection);
- Sample spiked with dissociated r-hFSH (4 μg by injection);
- Spiking solution (4 μg dissociated r-hFSH by injection)

Results are shown in the following Table 6:

**Table 6 : Results of sample preparations without Pluronic F68: area under peak**

| Spiking level | Area of spiking solution | % Recovery of areas | % Purity | % Recovery |
|---|---|---|---|---|
| 100% (2 μg/inj) | 922106 | N/A | 90% | 105% |
| 150% (3 μg/inj) | 1744753 | 189% | 89% | 104% |
| 200% (4 μg/inj) | 2702520 | 292% | 89% | 104% |

For the results obtained without the use of Pluronic F68 in the sample preparation, the recovery of the spike solution deviates 4-5% from the theoretical recovery (100%). For % recovery of areas, the results deviate significantly from the theoretical spiking level performed, due to a too low area of the spike solution.

It is believed that the different results obtained with and without Pluronic F68 can be explained by the adsorption of the test samples on the material used during sample preparation if Pluronic is not included in the assay solutions. The area under the peak of spiking solution obtained for the same spiking level (100%) is approximately two times greater when Pluronic F68 is present and avoids the adsorption.

The adsorption of free sub-units can be calculated by the difference between the area under the peak of the spiking solution with Pluronic F68 and the area of the spiking solution without Pluronic F68 at 2 μg per injection (100%) spiking level [i.e. subtracting the area under the peak at a spiking level of 100% without Puronic F68 (Table 6: 922106) from the area under the peak at a spiking level of 100% with Pluronic F68 (Table 5: 2037915)]. The difference corresponds to an area of 1’115’809. This area reflects the amount of dissociated sub-units adsorbed during sample preparation when Pluronic F68 is not present, and can be used to correct the area obtained in absence of Pluronic F68. The recoveries of area calculated taking into account this correction can be seen in table 7. The data are closer to the theoretical spiking level.

**Table 7: %Recovery of area with and without taking into account adsorption (in the absence of Pluronic F68)**

| Spiking level | %Recovery of areas taking into account adsorption | |
|---|---|---|
| | No | Yes |
| 3 μg/inj (150%) | 189% | 140% |
| 4 μg/inj (200%) | 292% | 187% |

The purity results presented in tables 5 and 6 differ without and with the introduction of Pluronic. This difference is also believed to be an effect of adsorption phenomenon. Percent purity is significantly more accurate when Pluronic F68 is used.

Several product batches were tested by SEC for purity without and with introduction of Pluronic F68 in sample preparation. The results can be seen in table 8:

**Table 8: %Recovery and %Purity results of drug product tested without and with Pluronic F68**

| Batch# | Without Pluronic | | With Pluronic | |
|---|---|---|---|---|
| | %Recovery | %Purity | %Recovery | %Purity |
| 17353066 | 98% | 89% | 96% | 94% |
| 17303019 | 101% | 91% | 100% | 97% |
| 17306019 | 104% | 91% | 97% | 98% |
| 17309029 | 100% | 90% | 100% | 97% |
| 17323049 | 101% | 92% | 98% | 97% |
| 17324049 | 98% | 94% | 97% | 99% |
| 17340089 | 100% | 93% | 99% | 97% |
| 17341089 | 101% | 91% | 98% | 98% |
| 17345119 | 100% | 90% | 100% | 96% |
| 17305019 | 105% | 88% | 99% | 101% |
| 17315039 | 99% | 91% | 101% | 95% |
| 17302019 | 100% | 87% | 99% | 96% |
| 17501019 | 106% | 86% | 99% | 96% |
| Mean | 101,0% | 90,2% | 98,7% | 97,0% |
| Standard deviation | 2,5% | 2,3% | 1,4% | 1,8% |
| CV % | 2,5% | 2,5% | 1,5% | 1,8% |

**Purity and recovery of spiking solution**

Based on the data presented in Table 8 above, it can be seen that the difference between the recovery without and with introduction of Pluronic is about 2%. This difference is statistically significant when an ANOVA at 95% confidence level is performed (p-value 0.008). In addition, when looking at the same table one can also see a statistically significant difference of approximately 7% in purity with and without introduction of Pluronic F68 (ANOVA p-value $1.2\ 10^{-8}$).

Furthermore, it can be seen from Table 8 that the CV% is lower when Pluronic F68 was included in the sample solution [2.5% without Pluronic F68 VS 1.5 and 1.8% with Pluronic F68]. A lower CV% indicates a lesser degree of variability between runs.

These statistical differences in purity and recovery of spiking solution can be explained by adsorption phenomenon which occurs during sample preparation when Pluronic F68 is not used (see the following Table 9).

In Table 9 and subsequent Tables, "Dimers and Aggregates" refers to aggregated FSH molecules and dimers of heterodimeric FSH that are generally considered to be undesirable.

**Table 9 : Area variability: area under peaks of heterodimeric FSH, free subunits and dimers and aggregates**

| Batch # | Total area of FSH spiking solution | | | | Total area of unspiked sample | | | | Dimers and aggregates area of sample | | | |
|---|---|---|---|---|---|---|---|---|---|---|---|---|
| | Area | | CV% | | Area | | CV% | | Area | | CV% | |
| Pluronic | No | Yes | No | Yes | No | Yes | No | Yes | No | Yes | No | Yes |
| 17353066 | 1409832 | 2658545 | 2,2 | 1,7 | 7377193 | 7767243 | 0,4 | 2,5 | 85841 | 90638 | 10,33 | 3,92 |
| 17303019 | 1438014 | 2513326 | 0,5 | 0,2 | 6136762 | 6312492 | 4,8 | 2,2 | 65913 | 64896 | 2,28 | 3,05 |
| 17306019 | 1438014 | 2513326 | 0,5 | 0,2 | 6029360 | 6474462 | 1,2 | 1,2 | 69387 | 65633 | 3,06 | 6,49 |
| 17309029 | 1121632 | 2380100 | 3,1 | 2,4 | 6554845 | 6906608 | 0,5 | 0,5 | 74694 | 83950 | 4,36 | 5,06 |
| 17323049 | 1300382 | 2429203 | 1,2 | 1,5 | 5692277 | 6157225 | 1,8 | 1,5 | 64801 | 67053 | 5,47 | 7,62 |
| 17324049 | 1300382 | 2429203 | 1,2 | 1,5 | 5856669 | 6129612 | 4,6 | 1,5 | 61088 | 58535 | 5,49 | 0,72 |
| 17340089 | 1449322 | 2564021 | 2,3 | 3,4 | 6316392 | 6577138 | 1,7 | 0,7 | 55831 | 64916 | 1,93 | 3,53 |
| 17341089 | 1449322 | 2564021 | 2,3 | 3,4 | 6311904 | 6658876 | 1,3 | 0,4 | 51410 | 66879 | 6,79 | 2,36 |

Table 9 : Area variability: area under peaks of heterodimeric FSH, free subunits and dimers and aggregates

| Batch # | Total area of FSH spiking solution | | | | Total area of unspiked sample | | | | Dimers and aggregates area of sample | | | |
|---|---|---|---|---|---|---|---|---|---|---|---|---|
| | Area | | CV% | | Area | | CV% | | Area | | CV% | |
| Pluronic | No | Yes | No | Yes | No | Yes | No | Yes | No | Yes | No | Yes |
| 17345119 | 1121632 | 2380100 | 3,1 | 2,4 | 5678718 | 6094436 | 0,5 | 0,3 | 61319 | 71681 | 5,86 | 0,98 |
| 17305019 | 1416915 | 2609854 | 3,5 | 0,8 | 6105716 | 6366397 | 3,4 | 1,3 | 81412 | 76271 | 8,77 | 1,96 |
| 17315039 | 1207140 | 2528909 | 7,4 | 5,7 | 6629111 | 7129130 | 1,4 | 0,6 | 69514 | 65511 | 1,74 | 3,77 |
| 17302019 | 1207140 | 2528909 | 7,4 | 5,7 | 6306795 | 6975041 | 3,8 | 0,6 | 97979 | 94241 | 5,28 | 8,59 |
| 17501019 | 1416915 | 2609854 | 3,5 | 0,8 | 7416153 | 7580193 | 1,1 | 2,1 | 69441 | 67857 | 6,4 | 6,1 |
| Mean | 1328972 | 2516105 | 2,9 | 2,3 | 6339377 | 6702219 | 2,0 | 1,2 | 69895 | 72159 | 5,2 | 4,2 |
| CV % | 9,5% | 3,5% | 76% | 81% | 8,7% | 8,1% | 77% | 63% | 18% | 15% | 50% | 59% |

Table 9 : Area variability (continued)

| Table 9 : Area variability (continued) | | | | | | | | |
|---|---|---|---|---|---|---|---|---|
| Batch # | Dissociated FSH area of spiked sample | | | | Total area of spiked sample | | | |
| | Area | | CV% | | Area | | CV% | |
| Pluronic | No | Yes | No | Yes | No | Yes | No | Yes |
| 17353066 | 2132665 | 3052448 | 2,5 | 1,08 | 8618890 | 9989197 | 0,92 | 0,41 |
| 17303019 | 1908454 | 2640090 | 4,5 | 0,43 | 7634720 | 8866597 | 1,52 | 0,83 |
| 17306019 | 1920007 | 2564678 | 1,2 | 0,81 | 7739564 | 8723677 | 1,12 | 0,58 |
| 17309029 | 1697129 | 2520633 | 6,0 | 2,01 | 7695502 | 9270545 | 2,16 | 1,48 |
| 17323049 | 1697821 | 2542802 | 1,8 | 1,14 | 7033218 | 8410312 | 1,14 | 1,09 |
| 17324049 | 1596022 | 2439971 | 6,4 | 2,61 | 7022864 | 8324957 | 2,32 | 0,81 |
| 17340089 | 1862023 | 2678583 | 3,2 | 1,27 | 7763745 | 9078480 | 0,79 | 1,49 |

**Table 9 : Area variability (continued)**

| Batch # | Dissociated FSH area of spiked sample | | | | Total area of spiked sample | | | |
|---|---|---|---|---|---|---|---|---|
| | Area | | CV% | | Area | | CV% | |
| Pluronic | No | Yes | No | Yes | No | Yes | No | Yes |
| 17341089 | 1977742 | 2642383 | 1,3 | 1,16 | 7846421 | 9034549 | 0,31 | 0,27 |
| 17345119 | 1616907 | 2555312 | 5,7 | 1,80 | 6808816 | 8480285 | 1,21 | 1,17 |
| 17305019 | 2044400 | 2479940 | 1,2 | 2,99 | 7863722 | 8851837 | 3,27 | 0,95 |
| 17315039 | 1722445 | 2785597 | 5,0 | 0,08 | 7721964 | 9750217 | 2,01 | 0,41 |
| 17302019 | 1943343 | 2681371 | 11,0 | 2,72 | 7543478 | 9363087 | 2,68 | 2,07 |
| 17501019 | 2354166 | 2847934 | 0,4 | 0,5 | 9364548 | 10138501 | 0,2 | 0,35 |
| Mean | 1882548 | 2648596 | 3,9 | 1,4 | 7742881 | 9098634 | 1,5 | 0,9 |
| CV % | 12% | 6,3% | 77% | 65% | 8,6% | 6,4% | 61% | 59% |

It can be seen from Table 9 that the precision (CV%) of the areas used for the purity and recovery of spiking solutions calculation is almost always better with Pluronic F68 than without the introduction of Pluronic F68 [lower CV% indicates improved precision].

The adsorption rate of dimers and aggregates, free sub-units and monomer are different as one can see when calculating the ratio mean area with Pluronic F68/ mean area without Pluronic F68. As can be seen in table 10, the %Area increase with Pluronic F68 is not constant depending on the area considered.

**Table 10: %Area increase with introduction of Pluronic F68**

| Area considered | %Area increase |
|---|---|
| Total area of spiking solution | 6% |
| Total area of unspiked sample | 89% |
| Dimers and aggregates areas of unspiked sample | 41% |
| Dissociated sub-units area of spiked sample | 3% |
| Total area of spiked sample | 18% |

To determine purity, spiking of free sub-units must be performed due to the fact that the free sub-units peak is not resolved from main peak during testing of sample preparation. The %Purity (or %Monomer) is expressed by the formula below:

$$\%\text{Purity} = \frac{A + B - C - D}{A}$$

Where:

A : Total area of sample without spike
B : Total area of spiking solution
C : Sub-units peak area of sample spiked
D : Dimers and aggregates in sample without spike

The acceptance criteria for recovery of spike is expressed as follows:

$$\%\text{Recovery} = \frac{E}{A + B}$$

Where:

A : Total area of sample without spike
B : Total area of spiking solution
E : Total area of spiked sample

Taking the above formulas into consideration, calculation of purity with and without introduction of Pluronic F68 can be performed. In addition, to take into consideration the effect of Pluronic F68, calculations can also be done with areas without introduction of Pluronic F68 taking into account the adsorption effect. The formulas will be the following :

$$\%\text{Purity corrected} = \frac{A \times Ra + B \times Rb - C \times Rc - D \times Rd}{A \times Ra}$$

$$\%\text{Recovery corrected} = \frac{E \times Re}{A \times Ra + B \times Rb}$$

Where:

A : Total area of sample without spike
B : Total area of spiking solution
C : Sub-units peak area of sample spiked
D : Dimers and aggregates in sample without spike
E : Total area of spiked sample
Rx: Ratio mean area X with Pluronic F68 divided by mean area X without Pluronic F68.

**EXAMPLE 3**

**Interferon beta-1a assay by RP-HPLC: Qualification of a One Standard point approach versus a standard curve approach**

This Example shows how, in the case of Interferon beta-1a assay by RP-HPLC, it was possible to modify a standard curve approach, hereinafter referred to as "current assay", to a One Standard Point approach by applying the improved method of this invention, that is, by using a Pluronic surfactant to avoid sample losses.

In the drug substance sample preparation for the improved method of the invention, Interferon beta-1a was diluted 1 to 7 using as the dilution buffer 0.05M sodium acetate containing 0.1% Poloxamer 188 (Pluronic F68) at pH 3.8 instead of 0.05M sodium acetate at pH 3.8 without Poloxamer.

The optimized RP-HPLC assay was qualified according to the ICH
Guidelines and the following characteristics were addressed:

- Range of linearity: The linearity was verified for the injection of Interferon-beta-1a in the range of 3.3 to 8.8 μg, with an intercept statistically not different from zero. These results support the One Standard Point approach.

- Precision: The precision of the optimized assay using the Drug Product Standard and the One Standard Point approach was compared to the precision obtained with the current assay using the Drug Substance Standard and the Standard curve approach. The overall precision obtained for one replicate with the optimized assay using Pluronic F68 (pooled CV of 1.2 % for all Drug Substances and Drug Products samples) was shown to be better than the precision of 1.9 % obtained for one replicate with the current assay, not using Pluronic F68.

- Specificity: the components that can be observed in the optimized assay are already present in the Drug Substance and Drug Product formulations analyzed in the current assay which was validated for specificity. The presence of Poloxamer 188 in the new Standard was demonstrated to not interfere in the optimized assay. As no additional components are introduced in the optimized assay, the optimized assay is therefore specific.

- Accuracy: The accuracy of the optimized assay was assessed by comparison of the results obtained with the optimized assay versus the results obtained with current assay, these last results being considered as the reference value. For this purpose, several Drug Substances and Drug Products (Liquid formulation containing HSA, Liquid formulation HSA free) were analyzed. In all cases, the ratio Optimized / Current Interferon-beta-1a assay was not statistically different from 100%, which demonstrates that the two Interferon beta-1a assays generate statistically equivalent results.

In conclusion, the method has a better precision than the current assay, and generates accurate results statistically equivalent to those obtained with the current assay.

This invention has been described with reference to examples of both quantitative determination of proteins and purity assessment of the same in both SEC and RP-HPLC chromatographic methods.

## Claims

**1.** A method of chromatographic analysis of a protein in a sample for quantifying the protein wherein the method comprises:

1) a step of preparing the protein sample to contain a poloxamer;
2) a step of performing a chromatography; and
3) a step of data manipulation to determine the quantity of the protein, wherein the quantity of the protein is determined using data from calibration with a standard.

**2.** A method of claim 1, further including the step of preparing a diluted sample for bringing the protein concentration to a level acceptable for the chromatographic system used.

**3.** A method of any of the preceding claims wherein the chromatography is size-exclusion chromatography (SEC) or reverse-phase HPLC (RP-HPLC).

**4.** A method of any of the preceding claims, wherein the protein on which analysis is carried out is a dimeric glycoprotein.

5. A method of any of the preceding claims wherein the protein on which analysis is carried out is FSH.

6. A method of any of the preceding claims 1 to 3, wherein the protein on which analysis is carried out is an interferon.

7. A method of claim 6 wherein the protein on which analysis is carried out is Interferon beta-1a.

8. A method of any of the preceding claims wherein the Poloxamer is Pluronic F68 (Poloxamer 188).

9. A method of claim 8 wherein Pluronic F68 is employed at a concentration of 100 μg/ml in ultra-pure water in the protein sample solution.

10. A method of claim 8 wherein Pluronic F68 is employed at a concentration of 0.1% in sodium acetate buffer at pH 3.8 in the protein sample solution.

11. The use of a poloxamer in chromatographic analysis for quantifying the protein in a protein sample.

## Patentansprüche

1. Verfahren zur chromatografischen Analyse eines Proteins in einer Probe zum Quantifizieren des Proteins, worin das Verfahren umfasst:

   1) einen Schritt der Herstellung der Proteinprobe, die ein Poloxamer enthält,
   2) einen Schritt des Durchführens einer Chromatografie und
   3) einen Schritt der Datenverarbeitung zum Bestimmen der Proteinmenge, worin die Proteinmenge unter Verwendung von Daten aus der Kalibrierung mit einem Standard bestimmt wird.

2. Verfahren nach Anspruch 1, das weiter den Schritt der Herstellung einer verdünnten Probe umfasst, um die Proteinkonzentration auf einen für das verwendete chromatografische System annehmbaren Wert zu bringen.

3. Verfahren nach einem der vorhergehenden Ansprüche, worin die Chromatografie Größenausschlusschromatografie (SEC) oder Umkehrphasen-HPLC (RP-HPLC) ist.

4. Verfahren nach einem der vorhergehenden Ansprüche, worin das Protein, an welchem die Analyse durchgeführt wird, ein dimeres Glycoprotein ist.

5. Verfahren nach einem der vorhergehenden Ansprüche, worin das Protein, an welchem die Analyse durchgeführt wird, FSH ist.

6. Verfahren nach einem der vorhergehenden Ansprüche 1 bis 3, worin das Protein, an welchem die Analyse durchgeführt wird, ein Interferon ist.

7. Verfahren nach Anspruch 6, worin das Protein, an welchem die Analyse durchgeführt wird, Interferon-beta-1a ist.

8. Verfahren nach einem der vorhergehenden Ansprüche, worin das Poloxamer Pluronic F68 (Poloxamer 188) ist.

9. Verfahren nach Anspruch 8, worin Pluronic F68 in einer Konzentration von 100 μg/ml in ultrareinem Wasser in der Proteinprobelösung verwendet wird.

10. Verfahren nach Anspruch 8, worin Pluronic F68 in einer Konzentration von 0,1 % in Natriumacetatpuffer bei pH 3,8 in der Proteinprobelösung verwendet wird.

11. Verwendung eines Poloxamers in der chromatografischen Analyse zum Quantifizieren des Proteins in einer Proteinprobe.

## Revendications

1. Procédé d'analyse chromatographique d'une protéine contenue dans un échantillon, pour la détermination quantitative de la protéine, le procédé comprenant:

   1) une étape de préparation de l'échantillon contenant la protéine pour qu'il contienne un poloxamère ;
   2) une étape d'exécution d'une chromatographie ; et
   3) une étape de traitement des données pour déterminer la quantité de la protéine, dans laquelle la quantité de la protéine est déterminée à l'aide de données provenant de l'étalonnage avec une substance de référence.

2. Procédé selon la revendication 1, incluant en outre l'étape de préparation d'un échantillon dilué pour porter la concentration de la protéine à une valeur acceptable pour le système chromatographique utilisé.

3. Procédé selon l'une quelconque des revendications précédentes, dans lequel la chromatographie est la chromatographie par exclusion en fonction de la taille (SEC) ou la HPLC à polarité de phases inversée (RP-HPLC).

4. Procédé selon l'une quelconque des revendications précédentes, dans lequel la protéine sur laquelle est effectuée l'analyse est une glycoprotéine dimère.

5. Procédé selon l'une quelconque des revendications précédentes, dans lequel la protéine sur laquelle est effectuée l'analyse est la FSH.

6. Procédé selon l'une quelconque des revendications 1 à 3, dans lequel la protéine sur laquelle est effectuée l'analyse est un interféron.

7. Procédé selon la revendication 6, dans lequel la protéine sur laquelle est effectuée l'analyse est l'interféron bêta-la.

8. Procédé selon l'une quelconque des revendications précédentes, dans lequel le poloxamère est le Pluronic F68 (poloxamère 188).

9. Procédé selon la revendication 8, dans lequel le Pluronic F68 est utilisé à une concentration dé 100 $\mu$g/ml dans de l'eau ultra-pure, dans la solution échantillon contenant une protéine.

10. Procédé selon la revendication 8, dans lequel le Pluronic F68 est utilisé à une concentration de 0,1 % dans du tampon acétate de sodium à pH 3,8, dans la solution échantillon contenant une protéine.

11. Utilisation d'un poloxamère dans l'analyse chromatographique pour la détermination quantitative de la protéine dans un échantillon contenant une protéine:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0530937 A
- DE 3917949


### Non-patent literature cited in the description


- **KATAKAM et al.** *Journal of Pharmaceutical Sciences,* 1995, vol. 84, 713-716